# FASCICULE DE BREVET EUROPEEN

(11) **EP 0 870 751 B1**
(45) Date de publication et mention de la délivrance du brevet: **18.06.2003**
(21) Numéro de dépôt: 98420060.0
(22) Date de dépôt: 08.04.1998
(51) Int. Cl.: C07C 51/31, C07C 55/14

(54) **Procédé d'oxydation d'hydrocarbures, d'alcools et/ou de cétones**
Verfahren zur Oxidation von Kohlenwasserstoffen, Alkoholen und/oder Ketonen
Process for the oxidation of hydrocarbons, alcohols and/or ketons

(30) Priorité: 10.04.1997 FR 9704637
(43) Date de publication de la demande: 14.10.1998
(73) Titulaire: Rhodia Polyamide Intermediates, 69192 Saint-Fons (FR)
(72) Inventeur: Costantini, Michel, 69003 Lyon (FR); Fache, Eric, 69300 Caluire et Cuire (FR)
(74) Mandataire: Esson, Jean-Pierre

(56) Documents cités:
- EP-A- 0 021 118
- EP-A- 0 784 045
- GB-A- 415 172
- GB-A- 1 086 951
- US-A- 3 987 100
- US-A- 4 902 827

## Description

La présente invention concerne l'oxydation par l'oxygène ou un gaz en contenant, d'hydrocarbures et/ou d'alcools et/ou de cétones en acides carboxyliques correspondants.

L'oxydation directe par l'oxygène des hydrocarbures, plus particulièrement des cycloalcanes, en présence d'un catalyseur, est un procédé qui a été étudié depuis longtemps. En effet, il y aurait des avantages évidents à éviter l'emploi d'un oxydant comme l'acide nitrique, utilisé dans une des étapes des procédés industriels actuels, ce qui épargnerait le traitement des oxydes d'azote générés.

Dans les nombreuses variantes d'un tel procédé d'oxydation catalytique par l'oxygène, le cobalt est le catalyseur le plus fréquemment préconisé.

Ainsi le brevet américain US-A-2 223 493, publié en décembre 1940, décrit l'oxydation d'hydrocarbures cycliques en diacides correspondants, en phase liquide comportant généralement de l'acide acétique, à une température d'au moins 60°C, à l'aide d'un gaz contenant de l'oxygène et en présence d'un catalyseur d'oxydation tel qu'un composé du cobalt.

Le brevet américain US-A-3 987 100 décrit un procédé de préparation de cyclohexanone et cyclohexanol par réaction de cyclohexane avec l'oxygène, en au moins trois étapes successives, à une température de 130°C à 180 °C et sous une pression d'environ 4 à 25 bar et en présence de très faibles quantités d'un système catalytique comprenant du cobalt et du chrome. Ce procédé est conduit sans solvant et ne permet pas de préparer directement l'acide adipique.

Le brevet anglais GB-A-1 086 951 décrit l'oxydation conjointe de mélanges de cyclohexanol et d'un aldéhyde, en phase liquide, par l'oxygène, en l'absence ou en présence d'un catalyseur comprenant au moins un composé d'un métal de transition. Ce procédé conduit à la cyclohexanone, à l'epsilon-caprolactone et à des acides dicarboxyliques à partir du cyclohexanol et à l'acide monocarboxylique correspondant à l'aldéhyde engagé.

Le brevet américain US-A-4 902 827, publié en février 1990, décrit un perfectionnement à l'oxydation par l'air du cyclohexane en acide adipique, en phase liquide comportant l'acide acétique, à une température de 80°C à 160°C et en présence d'un catalyseur d'oxydation comprenant un composé soluble du cobalt et un composé soluble du zirconium et/ou de l'hafnium.

Plus récemment, il a été préconisé dans le brevet EP-A-0 694 333 de mettre en oeuvre dans le cadre de l'oxydation des hydrocarbures par l'oxygène, un catalyseur comprenant un sel cobaltique et un sel ferrique.

Il s'avère néanmoins que les sélectivités obtenues avec les systèmes catalytiques utilisés dans les procédés antérieurs décrits précédemment doivent encore être améliorées.

C'est ce que se propose de réaliser la présente invention. Elle consiste plus précisément en un procédé d'oxydation d'hydrocarbure, d'alcool et/ou de cétone en acide carboxylique, à l'aide d'oxygène ou d'un gaz en contenant, en phase liquide dans un solvant choisi parmi les solvants protiques polaires et les solvants aprotiques polaires et en présence d'un catalyseur dissous dans le milieu réactionnel, caractérisé en ce que le catalyseur comprend au moins un composé soluble du cobalt et un composé soluble du chrome et en ce que les composés à oxyder ne contiennent pas d'aldéhyde.

Les hydrocarbures qui sont utilisés comme substrats de départ dans le procédé de l'invention sont plus particulièrement les alcanes, les cycloalcanes, les hydrocarbures alkyl-aromatiques, les alcènes et les cycloalcènes, ayant de 3 à 20 atomes de carbone.

Parmi ces hydrocarbures, les cycloalcanes, notamment ceux qui ont un cycle ayant de 5 à 12 atomes de carbone, sont certainement les plus importants, car leur oxydation conduit aux diacides carboxyliques et en plus faibles proportions aux cycloalcanols et cycloalcanones intermédiaires.

L'hydrocarbure le plus intéressant est le cyclohexane dont l'oxydation conduit à l'acide adipique, l'un des composés de base du polyamide 6-6.

Le présent procédé peut également être utilisé pour l'oxydation des alcools ou cétones intermédiaires, notamment les cycloalcanols et cyclohexanones ayant de 5 à 12 atomes de carbone, pour préparer les diacides carboxyliques correspondants. Dans ce qui suit, le procédé sera plus particulièrement décrit pour l'oxydation des hydrocarbures, essentiellement des cycloalcanes, et de manière privilégiée pour l'oxydation du cyclohexane.

Le catalyseur comprenant au moins un composé du cobalt et un composé du chrome permet de préparer directement l'acide adipique avec une bonne sélectivité, lorsque l'on réalise l'oxydation du cyclohexane ; cette capacité est évidemment très avantageuse.

Le catalyseur comprend au moins un composé du cobalt soluble dans le milieu réactionnel, choisi par exemple de manière non limitative parmi le chlorure de cobalt, le bromure de cobalt, le nitrate de cobalt, les carboxylates de cobalt comme l'acétate de cobalt tétrahydraté, le propionate de cobalt, l'adipate de cobalt, le glutarate de cobalt, le succinate de cobalt et les chélates de cobalt comme l'acétylacétonate de cobalt.

Le catalyseur comprend également au moins un composé du chrome soluble dans le milieu réactionnel, choisi par exemple de manière non limitative parmi le chlorure de chrome, le bromure de chrome, le nitrate de chrome, les carboxylates de chrome comme l'acétate de chrome, le propionate de chrome, l'adipate de chrome, le glutarate de chrome, le succinate de chrome et les chélates de chrome comme l'acétylacétonate de chrome.

Le rapport molaire entre le chrome et le cobalt du catalyseur peut varier dans de larges limites. On peut ainsi mettre en oeuvre des rapports molaires Cr/Co de 0,001 à 100, et de préférence de 0,01 à 10.

Le catalyseur peut être réalisé in situ en chargeant les composés du cobalt et du chrome dans le milieu réactionnel. Il peut également être préparé extemporanément par mélange desdits composés dans les proportions nécessaires pour obtenir le rapport molaire Cr/Co souhaité. Ce mélange est réalisé plus commodément dans un solvant, de préférence le solvant qui servira pour la réaction d'oxydation.

Le catalyseur mis en oeuvre dans le procédé de l'invention peut comprendre en outre au moins un composé du zirconium et/ou de l'hafnium soluble dans le milieu réactionnel. A titre d'exemples, les composés du zirconium et de l'hafnium peuvent être choisis de manière non limitative parmi le tétrachlorure de zirconium, le bromure de zirconium, le nitrate de zirconium, les carboxylates de zirconium comme l'acétate de zirconium, le propionate de zirconium, l'adipate de zirconium, le glutarate de zirconium, le succinate de zirconium, les chélates de zirconium comme l'acétylacétonate de zirconium, le chlorure d'hafnium, le bromure d'hafnium, le nitrate d'hafnium, les carboxylates d'hafnium comme l'acétate d'hafnium, le propionate d'hafnium, l'adipate d'hafnium, le glutarate d'hafnium, le succinate d'hafnium et les chélates d'hafnium comme l'acétylacétonate d'hafnium.

Le rapport molaire entre le zirconium et/ou l'hafnium et le cobalt du catalyseur est de 0 à 10, et de préférence de 0,001 à 5.

La quantité de catalyseur, exprimée en pourcentage pondéral d'élément cobalt et d'élément chrome par rapport au mélange réactionnel, se situe généralement entre 0,001 % et 5 % et de préférence entre 0,01 % et 2 %, sans que ces valeurs soient critiques. Il s'agit cependant d'avoir une activité suffisante tout en n'utilisant pas des quantités trop importantes d'un catalyseur qu'il faut ensuite séparer du mélange réactionnel final et recycler.

Il est avantageux de mettre en oeuvre également un composé initiateur de la réaction d'oxydation. Les initiateurs sont souvent des hydroperoxydes, comme par exemple l'hydroperoxyde de cyclohexyle ou l'hydroperoxyde de tertiobutyle. Ce sont également des cétones ou des aldéhydes, comme par exemple la cyclohexanone qui est l'un des composés formés lors de l'oxydation du cyclohexane ou l'acétaldéhyde. Généralement l'initiateur représente de 0,01 % à 20 % en poids du poids du mélange réactionnel mis en oeuvre, sans que ces proportions aient une valeur critique. L'initiateur surtout utile lors du démarrage de l'oxydation et lorsque l'on réalise l'oxydation du cyclohexane à une température inférieure à 120°C. Il peut être introduit dès le début de la réaction.

Le milieu réactionnel liquide contient un solvant au moins partiel de l'acide carboxylique dont la préparation est visée par la mise en oeuvre du procédé de l'invention. Ce solvant peut être de nature très variée dans la mesure où il n'est pas sensiblement oxydable dans les conditions réactionnelles. Il peut être choisi parmi les solvants protiques polaires et les solvants aprotiques polaires. Comme solvants protiques polaires, on peut citer par exemple les acides carboxyliques ne possédant que des atomes d'hydrogène primaires ou secondaires, en particulier les acides aliphatiques ayant de 2 à 9 atomes de carbone, les acides perfluoroalkylcarboxyliques tel que l'acide trifluoroacétique, les alcools tels que le tertiobutanol, les hydrocarbures halogénés tel que le dichlorométhane, les cétones telles que l'acétone. Comme solvants aprotiques polaires, on peut citer par exemple les esters d'alkyle inférieur (= radical alkyle ayant de 1 à 4 atomes de carbone) d'acides carboxyliques, en particulier des acides carboxyliques aliphatiques ayant de 2 à 9 atomes de carbone ou des acides perfluoroalkylcarboxyliques, la tétraméthylènesulfone (ou sulfolane), l'acétonitrile.

L'acide acétique est utilisé de préférence comme solvant de la réaction d'oxydation du cyclohexane. Il est commode de mettre en oeuvre un catalyseur dont les constituants cobalt et chrome soient sous la forme de composés dérivant de l'acide carboxylique utilisé comme solvant, dans la mesure où lesdits composés sont solubles dans le milieu réactionnel. Les acétates de cobalt et de chrome sont donc utilisés de préférence, notamment pour cette raison.

Le solvant, tel que défini précédemment, représente de 1 % à 99 % en poids du milieu réactionnel, de préférence de 10 % à 90 % et encore plus préférentiellement de 20 % à 80 %.

L'oxydation peut également être mise en oeuvre en présence d'eau introduite dès le stade initial du procédé.

La température à laquelle est réalisée la réaction d'oxydation est variable, notamment selon le substrat mis en oeuvre. Elle est généralement comprise entre 50°C et 200°C et de préférence entre 80 °C et 140°C.

La pression n'est pas un paramètre critique du procédé. Elle peut être inférieure, égale ou supérieure à la pression atmosphérique. Généralement elle se situera entre 0,1 MPa (1 bar) et 20 MPa (200 bar), sans que ces valeurs soient impératives.

On peut utiliser de l'oxygène pur, de l'air, de l'air enrichi ou appauvri en oxygène ou encore de l'oxygène dilué par un gaz inerte.

Les exemples qui suivent illustrent l'invention.

### EXEMPLE 1

Dans un autoclave de 125 ml en titane, muni de moyens de chauffage par collier chauffant, d'une turbine et de moyens d'introduction de gaz et de régulation de pression, on charge :
- 21,25 g (253 mmol) de cyclohexane
- 27,35 g d'acide acétique
- 0,26 g (2,65 mmol) de cyclohexanone
- 0,32 g (1,29 mmol de Co) d'acétate de Cobalt tétrahydraté
- 0,0331 g (0,1445 mmol de Cr) d'acétate de chrome.

Après fermeture du réacteur, on agite à 1000 tours par minute, on crée une pression d'air (100 bar à 20°C) et on chauffe. La température atteint 105°C dans la masse en 10 min et on maintient cette température pendant encore 50 min.

Après refroidissement et dépressurisation, le mélange réactionnel est constitué de deux phases liquides que l'on homogénéise par addition d'acide acétique.

Le mélange homogène ainsi obtenu est dosé par chromatographie en phase gazeuse.

On obtient les résultats suivants :
- taux de transformation (TT) du cyclohexane : 12,2 %
- sélectivité (ST) en cyclohexanol par rapport au cyclohexane transformé : 6,2 %
- sélectivité (ST) en cyclohexanone par rapport au cyclohexane transformé: 11,9 %
- ST en acide adipique par rapport au cyclohexane transformé : 67,3 %
- ST en acide adipique + cyclohexanone + cyclohexanol par rapport au cyclohexane transformé : 85,4 %
- rapport molaire acide adipique/total des diacides formés (acides adipique, glutarique et succinique) : 85,3 %
- ST en autres composés (butyrolactone, valérolactone acide hydroxyadipique, acide hydroxycaproïque) : 3,0 %

### ESSAI COMPARATIF 1

On répète l'exemple 1 dans le même appareillage et dans les mêmes conditions opératoires, mais en ne chargeant pas de composé du Cr et en utilisant une quantité d'acétate de Co tétrahydratée augmentée d'une quantité égale à la quantité molaire d'acétate de Cr de l'exemple 1.

On obtient les résultats suivants :
- taux de transformation (TT) du cyclohexane: 15,0 %
- ST en cyclohexanol par rapport au cyclohexane transformé : 14,7 %
- ST en cyclohexanone par rapport au cyclohexane transformé : 8,1 %
- ST en acide adipique par rapport au cyclohexane transformé : 54,7 %
- ST en acide adipique + cyclohexanone + cyclohexanol par rapport au cyclohexane transformé : 77,5 %
- rapport molaire acide adipique/total des diacides formés : 82,6 %
- ST en autres composés : 10,9 %

### ESSAI COMPARATIF 2

On répète l'essai comparatif 1 dans le même appareillage et dans les mêmes conditions opératoires, mais en utilisant une quantité d'acétate de Co tétrahydratée encore plus importante (1,45 g).

On obtient les résultats suivants :
- taux de transformation (TT) du cyclohexane : 13,2 %
- ST en cyclohexanol par rapport au cyclohexane transformé : 9,5 %
- ST en cyclohexanone par rapport au cyclohexane transformé : 8,5 %
- ST en acide adipique par rapport au cyclohexane transformé : 56,7 %
- ST en acide adipique + cyclohexanone + cyclohexanol par rapport au cyclohexane transformé : 74,7 %
- rapport molaire acide adipique/total des diacides formés : 81,2 %
- ST en autres composés : 12,2 %

### ESSAI COMPARATIF 3

On répète l'exemple 1 dans le même appareillage et dans les mêmes conditions opératoires, mais en remplaçant l'acétate de Cr par une même quantité molaire d'acétate de Fe.

On obtient les résultats suivants :
- taux de transformation (TT) du cyclohexane : 15,0 %
- ST en cyclohexanol par rapport au cyclohexane transformé : 9,3 %
- ST en cyclohexanone par rapport au cyclohexane transformé : 7,3 %
- ST en acide adipique par rapport au cyclohexane transformé : 58,9 %
- ST en acide adipique + cyclohexanone + cyclohexanol par rapport au cyclohexane transformé : 75,5 %
- rapport molaire acide adipique/total des diacides formés : 81,0 %
- ST en autres composés : 10,7 %

### ESSAI COMPARATIF 4

On répète l'exemple 1 dans le même appareillage et dans les mêmes conditions opératoires, mais en remplaçant l'acétate de Cr par une même quantité molaire d'acétate de Mn.

On obtient les résultats suivants :
- taux de transformation (TT) du cyclohexane : 13,9 %
- ST en cyclohexanol par rapport au cyclohexane transformé : 17,1 %
- ST en cyclohexanone par rapport au cyclohexane transformé : 8,1 %
- ST en acide adipique par rapport au cyclohexane transformé : 54,2 %
- ST en acide adipique + cyclohexanone + cyclohexanol par rapport au cyclohexane transformé : 79,4 %
- rapport molaire acide adipique/total des diacides formés : 83,4 %
- ST en autres composés : 9,8 %

### ESSAI COMPARATIF 5

Dans l'appareillage décrit dans l'exemple 1, on charge :
- 47,4 g d'acide acétique
- 0,25 g (2,54 mmol) de cyclohexanone
- 1,72 g (6,88 mmol de Co) d'acétate de Cobalt tétrahydraté
- 0,194 g (1,11 mmol de Fe) d'acétate de fer.

Après fermeture du réacteur, on agite à 1000 tours par minute, on crée une pression d'air (100 bar à 20°C) et on chauffe. La température atteint 105°C dans la masse en 10 min et on maintient cette température pendant encore 50 min.

Après refroidissement et dépressurisation, on rajoute alors 10,1 g de cyclohexane. Après fermeture du réacteur, on met une pression de 100 bar d'air et on chauffe à 105°C avec une agitation à 1000 tours/minute.

On laisse réagir pendant 1 heure à 105°C en maintenant constante la pression à l'aide d'une réserve d'oxygène pur.

Après refroidissement et dépressurisation du réacteur, le mélange réactionnel est constitué de deux phases liquides que l'on homogénéise par addition d'acide acétique.

Le mélange homogène ainsi obtenu est dosé par chromatographie en phase gazeuse.

On obtient les résultats suivants :
- taux de transformation (TT) du cyclohexane : 40,9 %
- ST en cyclohexanol par rapport au cyclohexane transformé : 9,6 %
- ST en cyclohexanone par rapport au cyclohexane transformé : 0 %
- ST en acide adipique par rapport au cyclohexane transformé : 66,8 %
- ST en acide adipique + cyclohexanone + cyclohexanol par rapport au cyclohexane transformé : 76,4 %
- rapport molaire acide adipique/total des diacides formés : 81,1 %
- ST en autres composés : 8,2 %

### EXEMPLE 2

On répète l'exemple 1 dans le même appareillage, dans les mêmes conditions de température et de pression et avec les mêmes réactifs, mais avec une durée de réaction de 115 min.

On obtient les résultats suivants :
- taux de transformation (TT) du cyclohexane : 15,0 %
- ST en cyclohexanol par rapport au cyclohexane transformé : 4,3 %
- ST en cyclohexanone par rapport au cyclohexane transformé : 0 %
- ST en acide adipique par rapport au cyclohexane transformé : 75,2 %
- ST en acide adipique + cyclohexanone + cyclohexanol par rapport au cyclohexane transformé : 79,5 %
- rapport molaire acide adipique/total des diacides formés : 83 %
- ST en autres composés : 5,0 %

### EXEMPLE 3

On répète l'exemple 2 dans le même appareillage, dans les mêmes conditions opératoires et avec les mêmes réactifs, mais en rajoutant 0,0049 g (0,01 mmol de Zr) d'acétylacétonate de Zr et avec une durée de réaction de 35 min.

On obtient les résultats suivants :
- taux de transformation (TT) du cyclohexane : 14,1 %
- ST en cyclohexanol par rapport au cyclohexane transformé : 4,8 %
- ST en cyclohexanone par rapport au cyclohexane transformé : 10,0 %
- ST en acide adipique par rapport au cyclohexane transformé : 67,1 %
- ST en acide adipique + cyclohexanone + cyclohexanol par rapport au cyclohexane transformé : 81,9 %
- rapport molaire acide adipique/total des diacides formés : 84,5 %
- ST en autres composés : 5,8 %

### EXEMPLE 4

On répète l'exemple 1 dans le même appareillage et dans les mêmes conditions opératoires, mais en utilisant 0,0672 g (0,29 mmol de Cr) d'acétate de Cr et avec une durée de réaction de 115 min.

On obtient les résultats suivants :
- taux de transformation (TT) du cyclohexane : 14,6 %
- ST en cyclohexanol par rapport au cyclohexane transformé : 4,5 %
- ST en cyclohexanone par rapport au cyclohexane transformé : 3,5 %
- ST en acide adipique par rapport au cyclohexane transformé : 72,9 %
- ST en acide adipique + cyclohexanone + cyclohexanol par rapport au cyclohexane transformé : 80,8 %
- rapport molaire acide adipique/total des diacides formés : 84,7 %
- ST en autres composés : 6,0 %

### EXEMPLE 5

On répète l'exemple 1 dans le même appareillage et dans les mêmes conditions opératoires, mais en utilisant 0,0033 g (0,0145 mmol de Cr) d'acétate de Cr et avec une durée de réaction de 60 min.

On obtient les résultats suivants :
- taux de transformation (TT) du cyclohexane : 14,0 %
- ST en cyclohexanol par rapport au cyclohexane transformé : 7,6 %
- ST en cyclohexanone par rapport au cyclohexane transformé : 14,7 %
- ST en acide adipique par rapport au cyclohexane transformé : 59,4 %
- ST en acide adipique + cyclohexanone + cyclohexanol par rapport au cyclohexane transformé : 81,7 %
- rapport molaire acide adipique/total des diacides formés : 83,7 %
- ST en autres composés : 6,5 %

## Revendications

1. Procédé d'oxydation d'hydrocarbure, d'alcool et/ou de cétone en acide carboxylique, à l'aide d'oxygène ou d'un gaz en contenant, en phase liquide dans un solvant choisi parmi les solvants protiques polaires et les solvants aprotiques polaires et en présence d'un catalyseur dissous dans le milieu réactionnel, **caractérisé en ce que** le catalyseur comprend au moins un composé soluble du cobalt et un composé soluble du chrome et **en ce que** les composés à oxyder ne contiennent pas d'aldéhyde.

2. Procédé selon la revendication 1, **caractérisé en ce que** l'hydrocarbure utilisé comme substrat de départ est choisi parmi les cycloalcanes qui ont un cycle ayant de 5 à 12 atomes de carbone, et est de préférence le cyclohexane.

3. Procédé selon la revendication 1, **caractérisé en ce que** l'alcool et/ou la cétone utilisés comme substrats de départ sont choisis parmi les cycloalcanols et cycloalcanones qui ont un cycle ayant de 5 à 12 atomes de carbone, et sont de préférence le cyclohexanol et/ou la cyclohexanone.

4. Procédé selon l'une des revendications 1 à 3, **caractérisé en ce que** le catalyseur comprend au moins un composé du cobalt soluble dans le milieu réactionnel, choisi parmi le chlorure de cobalt, le bromure de cobalt, le nitrate de cobalt,
les carboxylates de cobalt comme l'acétate de cobalt tétrahydraté, le propionate de
cobalt, l'adipate de cobalt, le glutarate de cobalt, le succinate de cobalt et les chélates de cobalt comme l'acétylacétonate de cobalt.

5. Procédé selon l'une des revendications 1 à 4, **caractérisé en ce que** le catalyseur comprend au moins un composé du chrome soluble dans le milieu réactionnel, choisi parmi le chlorure de chrome, le bromure de chrome, le nitrate de chrome, les carboxylates de chrome comme l'acétate de chrome, le propionate de chrome, l'adipate de chrome, le glutarate de chrome, le succinate de chrome et les chélates de chrome comme l'acétylacétonate de chrome.

6. Procédé selon l'une des revendications 1 à 5, **caractérisé en ce que** le rapport molaire entre le chrome et le cobalt du catalyseur est de 0,001 à 100, et de préférence de 0,01 à 10.

7. Procédé selon l'une des revendications 1 à 6, **caractérisé en ce que** le catalyseur comprend en outre au moins un composé du zirconium et/ou de l'hafnium soluble dans le milieu réactionnel, choisi parmi le tétrachlorure de zirconium, le bromure de zirconium, le nitrate de zirconium, les carboxylates de zirconium comme l'acétate de zirconium, le propionate de zirconium, l'adipate de zirconium, le glutarate de zirconium, le succinate de zirconium, les chélates de zirconium comme l'acétylacétonate de zirconium, le chlorure d'hafnium, le bromure d'hafnium, le nitrate d'hafnium, les carboxylates d'hafnium comme l'acétate d'hafnium, le propionate d'hafnium, l'adipate d'hafnium, le glutarate d'hafnium, le succinate d'hafnium et les chélates d'hafnium comme l'acétylacétonate d'hafnium.

8. Procédé selon la revendication 7, **caractérisé en ce que** le rapport molaire entre le zirconium et/ou l'hafnium et le cobalt du catalyseur est de 0 à 10, et de préférence de 0,001 à 5.

9. Procédé selon l'une des revendications 1 à 8, **caractérisé en ce que** la quantité de catalyseur, exprimée en pourcentage pondéral d'élément cobalt et d'élément chrome par rapport au mélange réactionnel, se situe entre 0,001 % et 5 % et de préférence entre 0,01 % et 2 %.

10. Procédé selon l'une des revendications 1 à 9, **caractérisé en ce que** le milieu réactionnel liquide contient un solvant choisi parmi les acides carboxyliques aliphatiques ayant de 2 à 9 atomes de carbone, les acides perfluoroalkylcarboxyliques, les alcools, les hydrocarbures halogénés, les cétones, les esters d'alkyle inférieur d'acides carboxyliques, de préférence des acides carboxyliques aliphatiques ayant de 2 à 9 atomes de carbone ou des acides perfluoroalkylcarboxyliques, la tétraméthylène sulfone (ou sulfolane), l'acétonitrile.

11. Procédé selon l'une des revendications 1 à 10, **caractérisé en ce que** le solvant utilisé est l'acide acétique.

12. Procédé selon l'une des revendications 1 à 11, **caractérisé en ce que** le solvant représente de 1 % à 99 % en poids du milieu réactionnel, de préférence de 10 % à 90 % et encore plus préférentiellement de 20 % à 80 %.

13. Procédé selon l'une des revendications 1 à 12, **caractérisé en ce que** la température à laquelle est réalisée la réaction d'oxydation est comprise entre 50°C et 200°C et de préférence entre 80 °C et 140°C.

14. Procédé selon l'une des revendications 1 à 13, **caractérisé en ce que** la pression à laquelle est réalisée la réaction d'oxydation est comprise entre 0,1 MPa (1 bar) et 20 MPa (200 bar).

## Patentansprüche

1. Verfahren zur Oxidation von Kohlenwasserstoff, Alkohol und/oder Keton zur Carbonsäure mit Hilfe von Sauerstoff oder eines Sauerstoff enthaltenden Gases in flüssiger Phase in einem Lösungsmittel, ausgewählt unter den polaren protischen Lösungsmitteln und den polaren aprotischen Lösungsmitteln sowie in Anwesenheit eines in dem Reaktionsmedium gelösten Katalysators, **dadurch gekennzeichnet, daß** der Katalysator mindestens eine lösliche Verbindung von Cobalt und eine lösliche Verbindung von Chrom umfaßt, und dadurch, daß die zu oxidierenden Verbindungen kein Aldehyd enthalten.

2. Verfahren nach Anspruch 1, **dadurch gekennzeichnet, daß** der als Ausgangssubstrat verwendete Kohlenwasserstoff unter den Cycloalkanen ausgewählt wird, die einen Ring mit 5 bis 12 Ringgliedern besitzen, und vorzugsweise Cyclohexan ist.

3. Verfahren nach Anspruch 1, **dadurch gekennzeichnet, daß** der als Ausgangssubstrat verwendete Alkohol und/oder das Keton unter den Cycloalkanolen und den Cycloalkanonen ausgewählt werden, die einen Ring mit 5 bis 12 Ringgliedern besitzen, und vorzugsweise Cyclohexanol und/oder Cyclohexanon sind.

4. Verfahren nach einem der Ansprüche 1 bis 3, **dadurch gekennzeichnet, daß** der Katalysator mindestens eine in dem Reaktionsmedium lösliche Verbindung von Cobalt umfaßt, ausgewählt unter Cobaltchlorid, Cobaltbromid, Cobaltnitrat, den Carboxylaten von Cobalt wie Cobaltacetat-Tetrahydrat, Cobaltpropionat, Cobaltadipat, Cobaltglutarat, Cobaltsuccinat und den Chelaten von Cobalt wie Cobaltacetylacetonat.

5. Verfahren nach einem der Ansprüche 1 bis 4, **dadurch gekennzeichnet, daß** der Katalysator mindestens eine in dem Reaktionsmedium lösliche Verbindung von Chrom umfaßt, ausgewählt unter Chromchlorid, Chrombromid, Chromnitrat, den Carboxylaten von Chrom wie Chromacetat, Chrompropionat, Chromadipat, Chromglutarat, Chromsuccinat und den Chelaten von Chrom wie Chromacetylacetonat.

6. Verfahren nach einem der Ansprüche 1 bis 5, **dadurch gekennzeichnet, daß** das molare Verhältnis des Katalysators zwischen Chrom und Cobalt 0,001 bis 100, vorzugsweise 0,01 bis 10 beträgt.

7. Verfahren nach einem der Ansprüche 1 bis 6, **dadurch gekennzeichnet, daß** der Katalysator mindestens eine in dem Reaktionsmedium lösliche Verbindung von Zirkonium und/oder Hafnium umfaßt, ausgewählt unter Zirkoniumtetrachlorid, Zirkoniumbromid, Zirkoniumnitrat, den Carboxylaten von Zirkonium wie Zirkoniumacetat, Zirkoniumpropionat, Zirkoniumadipat, Zirkoniumglutarat, Zirkoniumsuccinat, den Chelaten von Zirkonium wie Zirkoniumacetylacetonat, Hafniumchlorid, Hafniumbromid, Hafniumnitrat, den Carboxylaten von Hafnium wie Hafniumacetat, Hafniumpropionat, Hafniumadipat, Hafniumglutarat, Hafniumsuccinat und den Chelaten von Hafnium wie Hafniumacetylacetonat.

8. Verfahren nach Anspruch 7, **dadurch gekennzeichnet, daß** das molare Verhältnis des Katalysators zwischen Zirkonium und/oder Hafnium und Cobalt 0 bis 10 und vorzugsweise 0,001 bis 5 beträgt.

9. Verfahren nach einem der Ansprüche 1 bis 8, **dadurch gekennzeichnet, daß** die Menge an Katalysator, ausgedrückt in Gewichtsprozent Element Cobalt und Element Chrom zwischen 0,001 % und 5 % und vorzugsweise zwischen 0,01 % und 2 % beträgt, bezogen auf die Reaktionsmischung.

10. Verfahren nach einem der Ansprüche 1 bis 9, **dadurch gekennzeichnet, daß** das flüssige Reaktionsmedium ein Lösungsmittel enthält, ausgewählt unter den aliphatischen Carbonsäuren mit 2 bis 9 Kohlenstoffatomen, den Perfluoralkylcarbonsäuren, den Alkoholen, den halogenierten Kohlenwasserstoffen, den Ketonen, den niederen Alkylestern von Carbonsäuren, vorzugsweise den aliphatischen Carbonsäuren mit 2 bis 9 Kohlenstoffatomen oder den Perfluoralkylcarbonsäuren, Tetramethylensulfon (oder Sulfolan), Acetonitril.

11. Verfahren nach einem der Ansprüche 1 bis 10, **dadurch gekennzeichnet, daß** das verwendete Lösungsmittel Essigsäure ist.

12. Verfahren nach einem der Ansprüche 1 bis 11, **dadurch gekennzeichnet, daß** das Lösungsmittel 1 Gew.-% bis 99 Gew.-% des Reaktionsmediums darstellt, vorzugsweise 10 Gew.-% bis 90 Gew.-% und noch bevorzugter 20 Gew.-% bis 80 Gew.-%.

13. Verfahren nach einem der Ansprüche 1 bis 12, **dadurch gekennzeichnet, daß** die Temperatur, bei der die Oxidationsreaktion realisiert wird, zwischen 50 °C und 200 °C, vorzugsweise zwischen 80 °C und 140 °C beträgt.

14. Verfahren nach einem der Ansprüche 1 bis 13, **dadurch gekennzeichnet, daß** der Druck, bei der die Oxidationsreaktion realisiert wird, zwischen 0,1 MPa (1 bar) und 20 MPa (200 bar) beträgt.

## Claims

1. Process for the oxidation of hydrocarbon, alcohol and/or ketone into carboxylic acid, using oxygen or a gas containing it, in the liquid phase in a solvent chosen from polar protic solvents and polar aprotic solvents and in the presence of a catalyst dissolved in the reaction medium, **characterized in that** the catalyst comprises at least one soluble cobalt compound and a soluble chromium compound and **in that** the compounds to be oxidated do not contain any aldyhyde.

2. Process according to Claim 1, **characterized in that** the hydrocarbon used as starting substrate is chosen from cycloalkanes which have a ring having from 5 to 12 carbon atoms, and is preferably cyclohexane.

3. Process according to Claim 1, **characterized in that** the alcohol and/or the ketone used as starting substrates are chosen from cycloalkanols and cycloalkanones which have a ring having from 5 to 12 carbon atoms, and are preferably cyclohexanol and/or cyclohexanone.

4. Process according to one of Claims 1 to 3, **characterized in that** the catalyst comprises at least one cobalt compound which is soluble in the reaction medium, chosen from cobalt chloride, cobalt bromide, cobalt nitrate, cobalt carboxylates such as cobalt acetate tetrahydrate, cobalt propionate, cobalt adipate, cobalt glutarate, cobalt succinate and cobalt chelates such as cobalt acetylacetonate.

5. Process according to one of Claims 1 to 4, **characterized in that** the catalyst comprises at least one chromium compound which is soluble in the reaction medium, chosen from chromium chloride, chromium bromide, chromium nitrate, chromium carboxylates such as chromium acetate, chromium propionate, chromium adipate, chromium glutarate, chromium succinate and chromium chelates such as chromium acetylacetonate.

6. Process according to one of Claims 1 to 5, **characterized in that** the molar ratio between the chromium and the cobalt in the catalyst is from 0.001 to 100 and preferably from 0.01 to 10.

7. Process according to one of Claims 1 to 6, **characterized in that** the catalyst also comprises at least one zirconium and/or hafnium compound which is soluble in the reaction medium, chosen from zirconium tetrachloride, zirconium bromide, zirconium nitrate, zirconium carboxylates such as zirconium acetate, zirconium propionate, zirconium adipate, zirconium glutarate, zirconium succinate, zirconium chelates such as zirconium acetylacetonate, hafnium chloride, hafnium bromide, hafnium nitrate, hafnium carboxylates such as hafnium acetate, hafnium propionate, hafnium adipate, hafnium glutarate, hafnium succinate and hafnium chelates such as hafnium acetylacetonate.

8. Process according to Claim 7, **characterized in that** the molar ratio between the zirconium and/or hafnium and the cobalt in the catalyst is from 0 to 10 and preferably from 0.001 to 5.

9. Process according to one of Claims 1 to 8, **characterized in that** the amount of catalyst, expressed as a weight percentage of elemental cobalt and of elemental chromium relative to the reaction mixture, is between 0.001% and 5% and preferably between 0.01% and 2%.

10. Process according to one of Claims 1 to 9, **characterized in that** the liquid reaction medium contains a solvent chosen from aliphatic carboxylic acids having from 2 to 9 carbon atoms, perfluoroalkylcarboxylic acids, alcohols, halogenated hydrocarbons, ketones, lower alkyl esters of carboxylic acids, preferably aliphatic carboxylic acids having from 2 to 9 carbon atoms or perfluoroalkylcarboxylic acids, tetramethylene sulphone (or sulpholane) and acetonitrile.

11. Process according to one of Claims 1 to 10, **characterized in that** the solvent used is acetic acid.

12. Process according to one of Claims 1 to 11, **characterized in that** the solvent represents from 1% to 99% by weight of the reaction medium, preferably from 10% to 90% and even more preferably from 20% to 80%.

13. Process according to one of Claims 1 to 12, **characterized in that** the temperature at which the oxidation reaction is carried out is between 50°C and 200°C and preferably between 80°C and 140°C.

14. Process according to one of Claims 1 to 13, **characterized in that** the pressure at which the oxidation reaction is carried out is between 0.1 MPa (1 bar) and 20 MPa (200 bar).
